(19) **Europäisches Patentamt**

**European Patent Office**

**Office européen des brevets**

(11) **EP 1 029 546 A1**

(12) **EUROPEAN PATENT APPLICATION**
published in accordance with Art. 158(3) EPC

(43) Date of publication:
**23.08.2000 Bulletin 2000/34**

(21) Application number: **98950478.2**

(22) Date of filing: **30.10.1998**

(51) Int. Cl.[7]: **A61K 38/00**, A61K 38/58,
C07K 14/47, C07K 14/745,
C07K 14/81, C07K 1/14,
C07K 1/36

(86) International application number:
**PCT/JP98/04939**

(87) International publication number:
**WO 99/22753 (14.05.1999 Gazette 1999/19)**

(84) Designated Contracting States:
**AT BE CH CY DE DK ES FI FR GB GR IE IT LI LU
MC NL PT SE**
Designated Extension States:
**AL LT LV MK RO SI**

(30) Priority: **05.11.1997 JP 32034997**

(71) Applicant:
**YOSHITOMI PHARMACEUTICAL INDUSTRIES,
LTD.
Osaka-shi Osaka 541-0046 (JP)**

(72) Inventors:
• **GOTO, Takashi
Yoshitomi Pharmaceutical Ind. Ltd.,
Hirakata-shi, Osaka 573-1153 (JP)**

• **ASAHARA, Naomi
Yoshitomi Pharmaceutical Ind. ltd.
Hirakata-shi, Osaka 573-1153 (JP)**
• **OMIZU, Akimasa
Yoshitomi Pharmaceutical Ind. Ltd.
Hirakata-shi, Osaka 573-1153 (JP)**
• **UEMURA, Yahiro
International Reagents Corporation
Nishi-ku Kobe-shi, Hyogo 651 2241 (JP)**

(74) Representative:
**Helbing, Jörg, Dr. Dipl.-Chem. et al
Patentanwälte
von Kreisler-Selting-Werner,
Postfach 10 22 41
50462 Köln (DE)**

(54) **HEPARIN COFACTOR II PREPARATIONS AND PROCESS FOR PRODUCING THE SAME**

(57)   Heparin cofactor II (HCII)-containing preparations substantially free from at least one contaminant selected from among degrading factors, degraded HCII, polymerized HCII and coloring matters, in particular, HCII-containing preparations substantially free from degrading factors and degraded HCII; one of the preparations as stated above substantially free from any infective virus; and a process for producing these preparations which involves the step of effecting an operation selected from among hydrophobic chromatography, fractionation by using a water-soluble polymer, salting-out and affinity chromatography with the use of a basic amino acid as a ligand, preferably together with the step of filtration with the use of a porous hollow yarn.

Sequence Listing

SPECIMEN SEQUENCE LISTING

<110>    Yoshitomi Pharmaceutical Industries, Ltd.

<120>    Heparin Co-factor II Preparation and Process for the Production
         Thereof

<130>    09276

<150>    JP 9-320349
<151>    1997-11-05

<160>    4

<210>    1
<211>    20
<212>    DNA
<213>    Artificial Sequence

<220>
<223>    Oligonucleotide designed to act as sense primer for amplifying
         5' non-coding region of HCV genome.

<400>    1
gtgagtacac cggaattgcc        20


<210>    2
<211>    20
<212>    DNA
<213>    Artificial Sequence

<220>
<223>    Oligonucleotide designed to act as sense primer for amplifying
         5' non-coding region of HCV genome.

<400>    2
cacggtctac gagacctccc        20


<210>    3
<211>    20
<212>    DNA
<213>    Artificial Sequence

<220>
<223>    Oligonucleotide designed to act as antisense primer for amplifying 5' non-coding region of HCV genome.


<400>    3
acgaccgggt cctttcttgg        20



<210>    4
<211>    20
<212>    DNA
<213>    Artificial Sequence


<220>
<223>    Oligonucleotide designed to act as antisense primer for amplifying 5' non-coding region of HCV genome.


<400>    4
gcactcgcaa gcaccctatc        20

SEQUENCE LISTING

<110> Welfide Corporation

<120> Heparin Cofactor II Preparation and Process Therefor

<130>    09276

<150>    JP 9-320349
<151>    1997-11-05

<160>    4


<210> 1
<211> 20
<212> DNA
<213> Artificial Sequence

<220>
<221> Oligonucleotide designed to act as sense primer for amplifying
      5' non-coding region of HCV genome.

<400>    1
gtgagtacac cggaattgcc        20


<210>    2
<211> 20
<212> DNA
<213> Artificial Sequence

<220>
<221> Oligonucleotide designed to act as sense primer for amplifying
      5' non-coding region of HCV genome.

<400> 2
cacggtctac gagacctccc        20


<210> 3
<211> 20
<212> DNA
<213> Artificial Sequence

<220>
<223> Oligonucleotide designed to act as antisense primer for
      amplifying 5' non-coding region of HCV genome.

<400>    3
acgaccgggt cctttcttgg        20


<210> 4
<211> 20
<212> DNA
<213> Artificial Sequence

<220>
<223> Oligonucleotide designed to act as antisense primer for

```
amplifying 5' non-coding region of HCV genome.

<400> 4
gcactcgcaa gcaccctatc       20
```

## Description

### Technical Field

The present invention relates to a preparation containing a highly pure heparin cofactor II (hereinafter to be referred to as HCII) and a production method thereof. More particularly, the present invention relates to a preparation containing highly pure HCII, which is substantially free of a degrading factor, particularly a protease, and therefore, substantially free of a degraded HCII molecule, and to a production method thereof.

### Background Art

HCII is a single strand plasma glycoprotein having a molecular weight of about 72 kDa as measured by SDS polyacrylamide gel electrophoresis (SDS-PAGE). The nucleotide sequence of its cDNA has been already identified (*J. Biol. Chem., 257*, 2162-2169, 1982; *Nucleic Acids Res., 14*, 1073-1088, 1986). While the physiological action of HCII has not been fully elucidated, it is one of the anticoagulants that specifically inhibit thrombin. An other protein that shows an anti-thrombin action in blood, antithrombin III (hereinafter to be referred to as ATIII) has been well known. HCII acts on different sites from ATIII and is considered to function as a thrombin inhibitory substance in vascular media under vascular endothelial cells, rather than on vascular endothelial cells (*Thromb. Res., 62*, 409-419, 1991), and its applicability to the prophylaxis and treatment of various diseases associated with abnormal blood flow, sepsis and various organ disorders, various inflammations, and the like has been suggested (JP-A-9-176040).

HCII is purified from whole plasma, plasma fractions, cultures of recombinant host cells and the like. The contaminants to be removed during purification include plasma-derived components other than HCII and host-derived components, and polymerized and fragmented (degraded) HCII. Polymerized HCII shows no activity, and protein polymers are known to generally cause unwanted side effects, such as shock and hypotension, by its administration. Degraded HCII has been reported to contaminate a purified product from human plasma used as a starting material (*J. Biol. Chem., 260*, 2218-2225, 1985). HCII deficient in 67 residues from N-terminal is known to radically lower thrombin inhibition rate in the presence of heparin or dermatan sulfate (*Thoromb. Haemost., 74*, 1209-1214, 1995). In other words, degraded HCII may have low activity. In view of general properties of degraded protein, there is a possibility that degraded HCII shows antigenicity and that degraded HCII fails to maintain effect for a long time due to its short half life.

From the foregoing reports and preliminary knowledge of the present inventors, plasma or host cell derived proteases are particularly important factors that degrade HCII (i.e., degrading factor). For example, when HCII is purified from fraction I supernatant and fraction II + III supernatant, which are obtained from human plasma by Cohn's ethanol fractionation, and the like, degraded HCII fragments of 66 kDa and 60 kDa molecules are found to have been mixed therein in addition to 72 kDa molecule. These occur on cleavage of peptide bonds on the carboxyl side of lysine residues in HCII molecules. It has been postulated, therefore, that they occur as a result of the action of specific endopeptidase in the plasma.

Even if degraded HCII is completely separated and removed from intact HCII, the presence of protease in the final preparation results in degradation of HCII during storage. Although it is possible to add a protease inhibitor to inactivate proteases, it is desirable to eliminate proteases as completely as possible, because the preparation is clinically applied. Recombinant host cell-derived proteases should be removed from final preparation because of antigeniety. At present, however, conventional HCII purification methods are not able to completely separate HCII from proteases.

It is therefore an object of the present invention to provide an HCII-containing preparation substantially free of degrading factors, such as protease, and a production method thereof, as well as to provide an HCII-containing preparation substantially free of degraded HCII and a production method thereof. Another object of the present invention is to provide an HCII-containing preparation substantially free of polymerized HCII or coloring substances. A yet another object of the present invention is to provide a preparation containing HCII having a purity of not less than 98%, preferably a purity exceeding 99%, and a production method thereof. A further object of the present invention is to provide any of the above-mentioned HCII-containing preparations substantially free of infective viruses and a production method thereof.

### Disclosure of the Invention

The present invention is based on the finding that HCII and degrading factors can be efficiently separated by subjecting a solution containing HCII and degrading factors, such as protease, to one or more treatments from hydrophobic (interaction) chromatography, water soluble polymer treatment, salting out and affinity chromatography using basic amino acid as a ligand. Further, by applying a treatment such as affinity chromatography using immobilized heparin and anion exchange chromatography before or after the above step(s), and thereafter ultrafiltration and/or gel filtration chromatography, an HCII-containing solution can be obtained, which is substantially free of a degrading factor, polymerized or degraded HCII and a coloring substance, and which has an HCII purity of not less than 98%, preferably exceeding 99%. This HCII-containing solution is subjected to a virus removal or inactivation treatment. As a result, an HCII-containing preparation substantially free of infective viruses can be produced without exerting an adverse influence on HCII in the preparation.

Accordingly, the present invention provides an HCII-containing preparation substantially free of at least one of a degrading factor, degraded HCII, polymerized HCII and a coloring substance; preferably an HCII-containing preparation substantially free of a degrading factor and degraded HCII; and more preferably, the HCII-containing preparation further substantially free of polymerized HCII and/or a coloring substance. Moreover, the present invention provides an HCII-containing preparation having a purity of HCII contained in the preparation of not less than 98%, preferably exceeding 99%. Furthermore, the present invention provides any of the above-mentioned HCII-containing preparations, which is substantially free of infective viruses.

Another aspect of the present invention is a production method of an HCII-containing preparation, comprising a step of separating HCII and degrading factors from a solution containing HCII and the degrading factors, particularly a step comprising one or more steps from hydrophobic (interaction) chromatography, fractionation using a water soluble polymer, salting out and affinity chromatography using basic amino acid as a ligand. The present invention provides the production method further comprising a step for removing degraded HCII, particularly gel filtration. The present invention provides a production method of the above-mentioned HCII-containing preparation, which further comprises at least one step for virus removal or virus inactivation, which is selected from filtration, a heating treatment and a surfactant treatment, preferably filtration using a porous hollow fiber.

## Brief Description of the Drawing

Fig. 1 shows a profile of gel filtration chromatography of an HCII-containing fraction obtained by an immobilized heparin chromatography and anion exchange chromatography.
Fig. 2 shows SDS-polyacrylamide gel electrophoretic pattern (lanes 1 and 3:molecular weight markers, lane 2: non-reduced HCII, lane 4: reduced HCII) of effective HCII purified fraction obtained by gel filtration chromatography.
Fig. 3 shows high performance liquid chromatography analysis of effective HCII purified fraction obtained by gel filtration chromatography.

## Best Mode for the Embodiment of the Invention

The HCII contained in the HCII-containing preparation of the present invention is a protein that visually shows a single band at about 72 kDa molecular weight on SDS-PAGE, shows a single peak by high performance liquid chromatography, has an isoelectric point of about 5, and shows inhibitory action on thrombin, chymotripsin and cathepsin G. Therefore, the inventive HCII (hereinafter to be sometimes referred to particularly as an effective HCII to distinguish from polymerized or degraded HCII) is not necessarily limited to a molecule having a full length amino acid sequence (intact molecule), as long as it satisfies the above-mentioned properties.

An HCII-containing solution to be the starting material for the HCII-containing preparation of the present invention is not particularly limited as to its origin, as long as it contains an appropriate amount of HCII specified by the above-mentioned properties. It encompasses those derived from naturally occurring plasma and those derived from recombinant host cells genetically engineered to produce HCII. Those derived from plasma are exemplified by whole plasma, plasma fraction having high HCII activity, preferably supernatant I, supernatant II+III and fraction IV obtained by Cohn's ethanol fractionation. The biological origin of plasma is not particularly limited and may be plasma derived from human or cow, with preference given to plasma derived from human.

An HCII-containing solution derived from a recombinant host cell may be, for example, an HCII-containing fraction from a culture obtained by culturing a host cell transformed with a gene encoding HCII contained in the above-mentioned natural plasma. Examples thereof include culture broth, cell extract, culture supernatant and the like. The host cell is not particularly limited, but bacteria such as *Escherichia coli, Bacillus subtilis, lactobacillus* etc. and fungus such as yeast, plant and animal cells, insect cells etc. are preferable. In addition, a body fluid of a transgenic animal having a gene encoding HCII may be also used.

In the present invention, a degrading factor means a chemical and enzymological factor that causes fragmentation of HCII, exclusive of physical factors such as high temperature, low temperature, physical shear force and the like. Specific examples include protease, sugar chain or sialate degrading enzyme, strong acid, strong alkali and the like. In view of the origin of the HCII-containing solution to be the starting material, the primary degrading factor is a protease.

The production method of the present invention is characterized by a step for separating HCII and a degrading factor from a solution containing HCII and the degrading factor. This step is preferably hydrophobic (interaction) chromatography, fractionation using a water soluble polymer, salting out or affinity chromatography using a basic amino acid as a ligand. These treatments may be used alone or in combination.

The hydrophobic (interaction) chromatography comprises, for example, bringing a solution containing HCII and a degrading factor into contact with a carrier used for hydrophobic (interaction) chromatography at pH 6 - 9. The carrier for hydrophobic (interaction) chromatography is exemplified by $C_4$-$C_{18}$ alkyl type (e.g., butyl type, octyl type, octyldecyl type etc.), phenyl type and the like. Examples of the butyl type include butyl agarose, butyl polyvinyl (trademark: Butyl-Toyopearl, manufactured by Tosoh) and the like. Examples of the octyl type include octyl agarose and the like; examples of the octyldecyl type include octyldecyl agarose and the like; and examples of the phenyl type include phenyl cellulose (trademark: Phenyl celofine, manufactured by SEIKAGAKU CORPORATION) and the like. By this chromatography, HCII is recovered into an unadsorbed fraction and the degrading factor adsorbs to the carrier.

The fractionation using a water soluble polymer comprises adjusting the concentration of the water soluble polymer to 1 - 30% (w/v), preferably 3 - 20% (w/v), more preferably 6 - 12% (w/v), and adding the polymer to a solution containing HCII and a degrading factor. By this fractionation, HCII remains in the solution and the degrading factor sediments. The treated solution is centrifuged or filtered and the supernatant (filtrate) is recovered, whereby the degrading factor is separated from HCII. As used herein, the water soluble polymer means polyethylene glycol (PEG), nonionic surfactant and the like. PEG preferably has an average molecular weight of 4000 - 6000. Examples of the surfactant include polyoxyethylene (160) polyoxypropylene (30) glycol, polyoxyethylene (20) sorbitanmonooleate and the like.

The salting out comprises adding a salt of sodium, potassium, barium, ammonium and the like (e.g., chloride, sulfate, phosphate etc.) to a solution containing HCII and a degrading factor to a concentration of 0.05 - 0.25 M. Preferably, barium chloride is added to a concentration of 0.05 - 0.2 M. By this treatment, HCII remains in the solution and the degrading factor sediments. The treated solution is centrifuged or filtered and the supernatant (filtrate) is recovered, whereby the degrading factor is separated from HCII.

The affinity chromatography using a basic amino acid as a ligand comprises bringing a solution containing HCII and a degrading factor into contact, at pH 6 - 9, with a carrier for chromatography on which a basic amino acid (e.g., lysine, arginine etc.), preferably lysine, has been immobilized as a ligand. Examples of the carrier include polysaccharide, silica gel and an insoluble support made of fiber. To be specific, the carrier is agar, agarose, crosslinked agarose, cellulose, silica, nylon, hydrophilic vinyl polymer and the like. By this treatment, HCII is recovered into an unadsorbed fraction and the degrading factor adsorbs to the carrier.

To minimize generation of the degraded HCII, thereby to facilitate the subsequent purification and to increase recovery rate of HCII, the step for separating HCII from a degrading factor is preferably performed at an early stage of purification.

In the preferable embodiment of the present invention, a solution containing HCII and a degrading factor is first brought into contact with immobilized heparin to preferably bond HCII to heparin. The immobilized heparin is, for example, a heparin monomer bonded to a solid phase, such as polysaccharide, silica gel, an insoluble support made of fiber and the like. Examples of the support include agar, agarose, crosslinked agarose, cellulose, silica, nylon, hydrophilic vinyl polymer, and other known material in the pertinent field. Such immobilized heparin chromatography may be applied before and after the separation step of HCII from the degrading factor. By this treatment, the polymerized HCII and a part of the degraded HCII are separated into a fraction different from the monomer HCII (effective HCII) fraction and removed. In the present invention, the polymerized HCII means a polymer of HCII molecule, which has no physiological activity of HCII and has low affinity for heparin. More specifically, it is a dimer or a polymer of three or more monomers.

HCII is eluted from immobilized heparin by washing with a buffer solution having a salt concentration of about 0.05 - 0.2 M, preferably by repeat washing at stepwisely higher salt concentrations, and contact with a buffer having a salt concentration of about 0.05 - 1.0 M, preferably about 0.1 - 0.5 M, more preferably about 0.1 - 0.2 M. The salt component used to adjust ionic strength include sodium chloride, potassium chloride, ammonium sulfate, sodium thiocyanate, sodium sulfate and the like, with preference given to sodium chloride. The buffer is set to have a pH of 6 - 9.

In a more preferable embodiment of the present invention, the HCII-containing solution is further subjected to anion exchange chromatography. Examples of the anion exchanger include DEAE type [DEAE-sephadex, DEAE-cellulose, DEAE-sepharose, DEAE-polyvinyl (e.g., DEAE-Toyopearl) etc.] and QAE type [QAE-sephadex, QAE-cellulose, QAE-sepharose, QAE-polyvinyl (e.g., QAE-Toyopearl) etc.].

In the anion exchange chromatography, the anion exchanger is equilibrated and washed with 0.005 - 0.1 M citrate buffer (pH about 6 - 8), 0.005 - 0.1 M phosphate buffer (pH about 6 - 8), 0.005 - 0.1 M Tris-HCl buffer (pH 7 - 9) and the like. In addition, HCII is eluted from an anion exchanger by the use of a buffer for equilibration and washing, which contains sodium chloride at 0.05 - 0.5 M, preferably 0.2 - 0.25 M.

The order of the above-mentioned treatment for separating HCII from a degrading factor, an immobilized heparin chromatography and an anion exchange chromatography treatment is not particularly limited, and optionally determined. To prevent polymerization and degradation of HCII during the purification step as completely as possible, the above-mentioned treatments are desirably conducted at pH 6 - 9 and 4 - 20°C, unless particularly specified. Any buffer to be used for the above-mentioned steps preferably contains 0.001% - 1.0% (w/v) of a water soluble polymer (e.g., PEG4000 and the like) to prevent a polymer from being generated.

The HCII active fraction obtained by the above-mentioned purification may have been contaminated with degraded HCII molecules. In the present invention, the degraded HCII means a fragmented HCII molecule that gives a peak (shoulder) distinguishable from the peak of effective HCII by high performance liquid chromatography (hereinafter to be referred to as HPLC, the conditions of HPLC defined below). Therefore, the degraded HCII is preferably separated and removed by applying an HCII active fraction to a separation step based on the difference in molecular weights by ultrafiltration and/or gel filtration chromatography and the like.

An ultrafiltration membrane to be used for ultrafiltration is free of any particular limitation as long as it has a pore size that permits passage of degraded HCII alone but the effective HCII. Examples thereof include acetylcellulose, acryl-nitrile copolymer, aromatic nylon, polysulfone, vinylidene polyfluoride, polyimide resin membrane and the like. The membrane can take various forms such as a tubular membrane, a flat membrane, spiral module, hollow fiber module and the like. Since the pore size of the ultrafiltration membrane is not always the same, when the molecular weight of the degraded HCII to be removed does not differ greatly from that of the effective HCII, the effective HCII is preferably concentrated first by ultrafiltration and subjected to gel filtration chromatography.

The gel to be used for gel filtration chromatography may be, for example, crosslinked dextran beads (e.g., Sephadex), crosslinked polyacrylamide beads (e.g., Bio Gel), agarose gel (e.g., Sepharose), dextran acrylamide (e.g., Sephacryl) and the like. A 0.01 - 0.05 M citrate or phosphate buffer (pH about 6 - 9) containing 0 - 0.5 M, preferably 0.1 - 0.2 M, sodium chloride is used for gel filtration.

To prevent polymerization and degradation of HCII as completely as possible, the above-mentioned ultrafiltration and gel filtration chromatography are desirably conducted at pH 6 - 9 and 4 - 20°C. Any buffer to be used for the above-mentioned steps preferably contains 0.001% - 1.0% (w/v) of a water soluble polymer (e.g., PEG4000 and the like) to prevent a polymer from being generated.

The level of purification of HCII by each of the above-mentioned treatments is monitored using the protein amount, that is determined by the measurement of absorbance at wavelength 280 nm ($A_{280}$), and specific activity of HCII as indices. The activity of HCII can be determined by the method to be detailed in the following examples using thrombin activity inhibitory action as an index.

The purified HCII-containing solution obtained by the above-mentioned treatments is formulated into a preparation by a method known *per se* to give a preparation containing highly pure HCII. In doing so, where necessary, pharmaceutically acceptable carrier, additive and the like are added, and heating, sterilization, filtration for sterilization, virus inactivation, virus removal, dispensing, lyophilization and the like are applied according to conventional methods.

The purified HCII-containing solution of the present invention is preferably further subjected to virus inactivation and/or removal treatment step(s) to make the solution substantially free of infective viruses. As used herein, by substantially free of viruses is meant that the virus has been inactivated to the degree that the virus infection potency ($TCID_{50}$) is below detection limit or the virus has been removed to the degree that the viral genome amount is below 1 PCR unit (minimum nucleic acid amount detectable by PCR described in *Transfusion, 32*, 824-828, 1992).

The method of virus inactivation may be any that is conventionally known. For example, liquid heating, dry heating, treatment by surfactant and the like can be applied. It is preferable that a method exerting the least possible adverse influence on physiological activity of HCII, such as degeneration, degradation, polymerization and the like of HCII, be selected. Since HCII is unstable to heat, liquid heating, dry heating, treatment by surfactant and the like in the presence of a suitable stabilizer (e.g., acidic amino acid and the like) are more preferable.

A virus removal method preferably involves use of a virus removable membrane, namely, filtration using a membrane having a pore size that permits passage of HCII molecule but a viral particle. The virus removable membrane can take various forms such as a tubular membrane, a plane membrane, hollow fiber and the like, with preference given to a porous hollow fiber.

The porous hollow fiber is a tubular yarn having a number of pores that penetrate the surrounding wall from the hollow part inside the hollow fiber to the outside. This circumference wall acts as a membrane for filtration. The pores in the circumference wall of the porous hollow fiber to be used in the present invention have an average pore size of 1 nm - 100 nm, preferably 10 nm - 50 nm, more preferably 15±2 nm. This range of pores can remove contaminant viruses from the HCII-containing solution. The hollow part of the porous hollow fiber has an inner diameter of preferably 330±30 μm, and the thickness of the circumference wall (membrane thickness) is preferably 27±3 μm.

While the material forming the porous hollow fiber is not particularly limited, regenerated cellulose is preferably used. The porous hollow fiber made from regenerated cellulose is preferably prepared from cellulose copper ammonia solution by micro phase separation method (*Am. Chem. Soc., 9*, 197-228, 1985).

The porous hollow fiber is preferably used in the form of a module. For example, porous hollow fibers are bundled in parallel in great numbers, packed in a cartridge, and integrated with an adhesive.

The temperature of the HCII-containing solution is 4°C - 50°C, preferably 4°C - 20°C, during filtration. The filtration pressure is 0.1 kgf/cm$^2$ - 1 kgf/cm$^2$, preferably 0.4 kgf/cm$^2$ - 0.9 kgf/cm$^2$. Preferably, HCII concentration is set to 0.01 - 10% (w/v), more preferably 0.1 - 4% (w/v), to efficiently filter the HCII-containing solution.

The methods of filtration include a cross flow filtration method (circulation) wherein the solution is filtered with a strain rate and a dead-end filtration method (non-circulation) wherein the solution is filtered without a strain rate. Either method is preferably used while applying an air pressure.

The above-mentioned virus inactivating treatment and virus removal treatment may be used alone to substantially remove infective viruses, but combination of the bath is more preferable. The order of the treatment is not particularly limited but the virus inactivating treatment preferably precedes the virus removal treatment.

The HCII-containing preparation obtained by the above-mentioned steps is characterized by being substantially free of at least one of a degrading factor, degraded HCII, polymerized HCII and a coloring substance. In the present invention, the coloring substance means a contaminant that shows a specific absorption at 410 nm, such as haptoglobin, transferrin and the like. As used herein, by being substantially free of a degrading factor is meant that degraded HCII is not detected by HPLC (G3000SW$_{XL}$) after incubation in a 0.1 M sodium phosphate buffer at pH 8 and 25°C for 48- 120 hours. As used herein, by being substantially free of degraded (or polymerized) HCII is meant that a peak of degraded (or polymerized) HCII is not detected by HPLC (G3000SW$_{XL}$) wherein the conditions of HPLC are as follows.

column: G3000SW$_{XL}$ (⌀7.8 mm×30 cm; manufactured by Tosoh)
eluent: 0.1 M sodium acetate, 0.3 M sodium chloride, 0.1% sodium azide (pH 6.5)
flow rate:1.0 mL/min
detection wavelength:280 nm
detection sample injection amount:50 μL (A$_{280}$ = about 1 (in the range of minimum 0.2 - maximum 2))

Further, by being substantially free of a coloring substance is meant that the color index (C.I.) to be defined in the example below is not more than 50. Preferably, the HCII containing preparation of the present invention does not contain substantially a degrading factor or degraded HCII; more preferably, the HCII-containing preparation is further substantially free of one or both of polymerized HCII and a coloring substance.

In a particularly preferable embodiment, the HCII preparation of the present invention contains effective HCII purified to have an HCII purity relative to the total protein of not less than 98%, particularly not less than 99.9%.

The present invention is explained in detail by referring to Examples in the following, but the present invention is not limited by these examples. Any buffer to be used in the purification step in the examples to be mentioned below contains 0.01% - 0.1% (w/v) of a water soluble polymer (e.g., PEG4000 and the like) to prevent polymerization of HCII.

**Comparative Example 1: purification of HCII from human plasma fraction - (I)**

(1) Immobilized heparin treatment

Supernatant II+III obtained by Cohn's fractionation was subjected to affinity chromatography using heparin gel, and elution with a buffer containing 0.4 M NaCl. The obtained fraction (HCII purity about 1%; hereinafter to be referred to as a crude purification product) was applied to a column (⌀5×2.5 cm) of heparin gel equilibrated with 20 mM phosphate buffer (pH 8.0) at 4°C (70[A$_{280}$]/mL gel), and the adsorbed fraction was eluted with a 20 mM phosphate buffer (pH 8.0) containing 0.1 - 0.15 M NaCl. As a result, an HCII-containing fraction having a 7.5 to 9.6 times higher specific activity was obtained from the crude purification product. The activity of HCII was measured in the following manner. That is, to the diluted solution containing a sample were added 40 mM Tris buffer (50 μL, pH 8.56) containing 60 mM EDTA, 0.2% HSA and 0.1mg/mL dermatan sulfate, and a 1.0 U/mL throwbin solution (100 μL) containing 0.05% bovine serum albumin and 0.05% PEG6000, and the mixture was incubated at 37°C for 5 min. Thereto was added a 0.95 mg/mL H-D-phenylalanyl-L-pipecolyl-L-arginyl-p-nitroanilide dihydrochloride solution (100 μL) and the mixture was incubated at 37°C for 5 min. 2% citric acid (1 mL) was added to stop the reaction and absorbance at 405 nm (A$_{405}$) was measured. The HCII activity was calculated from a calibration curve depicted using the partially purified product prepared by a conventional method as a standard product [protein concentration 0.1 mg/mL determined from absorption coefficient:E$^{1\%}$=5.93 (*Arch. Biochem. Biophys., 259*, 331-340, 1997) and A$_{280}$ was taken as 1U].

(2) Anion exchange chromatography

The HCII-containing fraction obtained in (1) was applied to a QAE-polyvinyl column (⌀5×2.5cm) equilibrated with 40 mM phosphate buffer (pH 8.0) at 4°C at 12[A$_{280}$]/mL gel. The adsorbed fraction was eluted with 40 mM phosphate buffer (pH 8.0) containing 0.15 - 0.40 M NaCl (stepwise gradient by 0.05 M). The HCII activity showing a specific activity of 15.4 $U/A_{280}$ was obtained at a yield of 92% from the fraction eluted with a buffer containing 0.20 - 0.25 M NaCl.

Calculating from the crude purification product, an HCII-containing fraction having a 67 to 91 times higher specific activity was obtained at a yield of 71%. This HCII-containing fraction was analyzed by SDS-PAGE (20 mA/gel, about 1.5 hours) using 10 - 20% gradient gel (manufactured by G10 Daiichi Pure Chemicals). The gel was stained using a quick staining kit (manufactured by Waco Pure Chemicals Co., Ltd.). As a result, a main band was detected at 72 kDa which corresponded to the effective HCII. Along therewith, a 66 kDa band corresponding to degraded HCII deficient in N-terminal 42 amino acid residues and a 35 kDa band of unidentified components were detected. This HCII-containing fraction was left standing in a cold room. The degradation of HCII sometimes advanced further to make detection of 72 kDa molecule ultimately unattainable. Therefore, it was clarified that the immobilized heparin chromatography and anion exchange chromatography were not able to remove an HCII degrading factor, particularly protease.

(3) Gel filtration chromatography

The HCII-containing fraction obtained in (2) was concentrated using a 10 kDa cut ultrafiltration membrane to a protein concentration of 20 mg/mL. The fraction was applied to a Superdex 200pg column ($\varnothing 1.6 \times 60$ cm; manufactured by Pharmacia) for gel filtration chromatography, in an amount corresponding to 1% volume (1.2 mL) of the column, to allow elution under the following conditions.

buffer :0.15 M NaCl-containing 20 mM phosphate buffer (pH 8.0)
flow rate:10 hr/column
temperature:4°C

As a result, the peak of HCII activity included a peak of the main fraction (effective HCII purified fraction) corresponding to 72 kDa molecule and a peak of lower molecule fraction (degraded HCII-containing fraction). The specific activity of the effective HCII purified fraction was 16.8 $U/A_{280}$. As a result of SDS-PAGE analysis of the effective HCII fraction, a single band was detected at 72 kDa, and a single peak was also detected by HPLC ($G3000SW_{XL}$). Therefore, the purified fraction was shown to be substantially free of degraded and polymerized HCII molecules. The color index [defined to be $C.I.=A_{410}/A_{280} \times 1000$] of this purified fraction was 12.3, and was substantially free of coloring substance such as haptoglobin, transferrin and the like. However, when the effective HCII purified fraction was left standing in a cold room or at room temperature, the degradation advanced further as in the above-mentioned (2). Therefore, it was found that a degrading factor (protease) could not be removed completely by gel filtration, either.

**Comparative Example 2: purification of HCII from human plasma fraction - (II)**

(1) Immobilized heparin treatment

In the same manner as in Comparative Example 1 (1) except the buffer used was 20 mM Tris-HCl buffer (pH 8.5), the amount of the HCII crude purification product to be applied was 30 $[A_{280}]$/mL gel, and eluent was 20 mM Tris-HCl buffer (pH 8.5) containing 0.1 M NaCl, an immobilized heparin chromatography was conducted. As a result, an HCII-containing fraction having a specific activity that was 13.6 - 19.3 times as high as that of the crude purification product was obtained.

(2) Anion exchange chromatography

In the same manner as in Comparative Example 1 (2) except the adsorbed fraction was eluted with 40 mM phosphate buffer (pH 8.0) containing 0.15 - 0.45 M NaCl (stepwise gradient by 0.05 M), anion exchange chromatography was performed. As a result, the HCII activity showing a specific activity of 17.4 $U/A_{280}$ was obtained at a yield of 95% from the fraction eluted with a buffer containing 0.20 - 0.25 M NaCl.

Calculating from the crude purification product, an HCII-containing fraction having a 76 to 102 times higher specific activity was obtained at a yield of 66%. This HCII-containing fraction was analyzed by SDS-PAGE in the same manner as in Comparative Example 1 (2). As a result, a main band was detected at 72 kDa which corresponded to the effective HCII. Along therewith, a 66 kDa band corresponding to degraded HCII deficient in N-terminal 42 amino acid residues and a 35 kDa band of unidentified components were detected. This HCII-containing fraction was left standing in a cold room. The degradation of HCII sometimes advanced further to make detection of 72 kDa molecule ultimately unattainable.

(3) Gel filtration chromatography

The HCII-containing fraction obtained in (2) was concentrated using a 10 kDa cut ultrafiltration membrane to a protein concentration of 20 mg/mL. The fraction was applied to a Superdex 200pg column (Ø1.6×60 cm; manufactured by Pharmacia) for gel filtration chromatography, in an amount corresponding to 1% volume (1.2 mL) of the column, to allow elution under the following conditions.

    buffer :0.15 M NaCl-containing 20 mM phosphate buffer (pH 8.0)
    flow rate:10 hr/column
    temperature:4°C

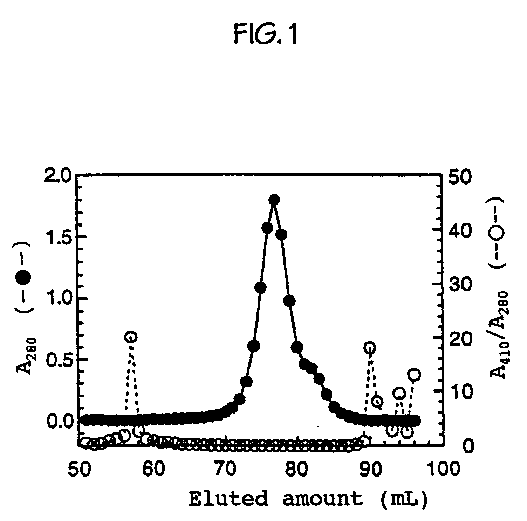

As a result, the peak of HCII activity included a peak of the main fraction (effective HCII purified fraction) corresponding to 72 kDa molecule and a peak of lower molecule fraction (degraded HCII-containing fraction). The effective HCII purified fraction showed the same maximum peak at $A_{280}$ (Fig. 1). The specific activity of the effective HCII purified fraction was 17.1 $U/A_{280}$. As a result of SDS-PAGE analysis of the effective HCII purified fraction, a single band was detected at 72 kDa, and a single peak was also detected by HPLC (G3000SW$_{XL}$). Therefore, the purified fraction was shown to be substantially free of degraded and polymerized HCII molecules. The color index [defined to be C.I.=$A_{410}/A_{280}\times1000$ ] of this purified fraction was 4.2, and the fraction was substantially free of coloring substances such as haptoglobin, transferrin and the like. The proportion of HCII in the obtained HCII purified fraction to the total protein amount was not less than 99.9%. However, when the effective HCII purified fraction was left standing in a cold room or at room temperature, the degradation advanced further as in Comparative Example 1.

**Example 1: Removal of degrading factor by hydrophobic chromatography - (I)**

A part of the HCII-containing fraction obtained in Comparative Example 2, step (2) was passed through Butyl Toyopearl (manufactured by Tosoh) at 4°C, which had been equilibrated with 40 mM phosphate buffer (pH 7.1) containing NaCl at not less than 1.5 M, and incubated at 25°C for not less than 48 hr. When this solution was analyzed by HPLC (G3000SW$_{XL}$), an increase in the degraded HCII content was not found. Thus, the degrading factor was found to have been removed by the above-mentioned hydrophobic (interaction) chromatography.

**Example 2: Removal of degrading factor by hydrophobic chromatography - (II)**

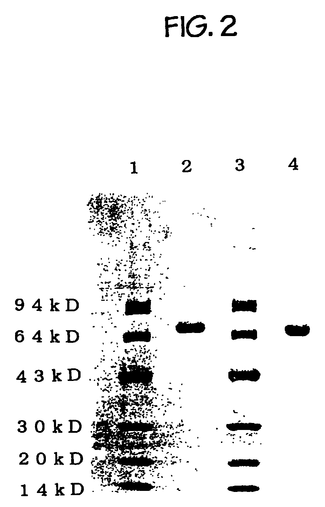

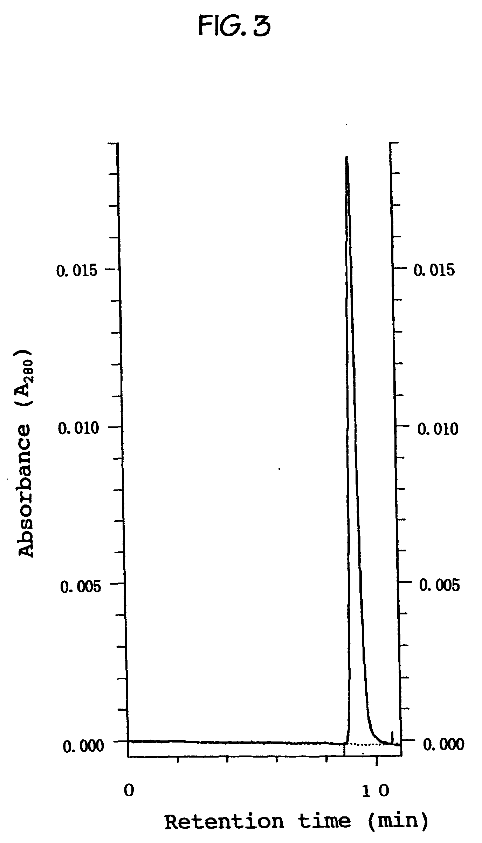

In the same manner as in Example 1 except that phenyl Sepharose (manufactured by Pharmacia) was used as a carrier for hydrophobic (interaction)chromatography and phosphate buffer (pH 8) containing NaCl at not less than 0.1 M was used for equilibration, hydrophobic (Interaction) chromatography was performed. The obtained unadsorbed fraction was treated in completely the same manner as in Example 1 and assayed for degradation of HCII, but degradation was not observed in result. Further, it was purified by gel filtration chromatography. As a result, a single band was detected at 72 kDa on SDS-PAGE (Fig. 2), and a single peak was also detected by HPLC (Fig. 3). Therefore, the purified fraction was proved to be substantially free of not only a degrading factor but degraded and polymerized HCII.

**Example 3: Removal of degrading factor by affinity chromatography using lysine as ligand**

A part of the HCII-containing fraction obtained in Comparative Example 2, step (2) was passed through a crosslinked agarose support using, as a ligand, lysine equilibrated with 40 mM phosphate buffer (pH 7.6), at 4°C. The obtained unadsorbed fraction was treated in completely the same manner as in Example 1 and assayed for degradation of HCII, but degradation was not observed in result, either. Therefore, it was clarified that the degrading factor was removed by the above-mentioned affinity chromatography.

**Example 4: Removal of degrading factor by fractionation using water soluble polymer - (I)**

To a part of the HCII-containing fraction obtained in Comparative Example 2, step (2) was added PEG4000 or polyoxyethylene (160) polyoxypropylene (30) glycol (trademark: pluronic F68) to a concentration of 6 - 12% (w/v), and the mixture was left standing for a given period of time. The supernatant was recovered by centrifugation. The supernatant was applied to Comparative Example 2, step (3), treated in the same manner as in Example 1, and assayed for degradation of HCII, but degradation was not observed in result, either. Therefore, it was clarified that the degrading factor was removed by the above-mentioned fractionation using a water soluble polymer or nonionic surfactant.

**Example 5: Removal of degrading factor by salting out**

To a part of the HCII-containing fraction obtained in Comparative Example 2, step (2) was added 1M barium chloride solution to a concentration of 5 - 20% (v/v), and the mixture was left standing for a given period of time. The supernatant was recovered by centrifugation. The supernatant was applied to Comparative Example 2, step (3), treated in the same manner as in Example 1, and assayed for degradation of HCII, but degradation was not observed in result, either. Therefore, it was clarified that the degrading factor was removed by the above-mentioned salting out.

**Example 6: Removal of virus by filtration using porous hollow fiber**

As the porous hollow fiber, a module using BMM (Bemberg Microporous Membrane, manufactured by Asahi Chemical Industry Co., Ltd.) was used. BMM was a porous hollow fiber made from regenerated cellulose as a starting material and obtained by copper ammonia method. As the BMM module, Planova$^®$ 15 (manufactured by Asahi Chemical Industry Co., Ltd.) was used, which had a multi structure of 150 layers or more for the circumference wall that acted as a membrane. The specification of the porous hollow fiber in the module was as follows.

    average pore size:15 nm$\pm$2 nm
    hollow fiber inner diameter:330$\pm$30 $\mu$m
    membrane thickness:27$\pm$3 $\mu$m

This module consists of the above-mentioned porous hollow fiber, an autoclavable polycarbonate plastic container, and a polyurethane adhesive to integrate these. This module has been autoclave-sterilized and filled with distilled water for injection. The safety of the various materials consisting Planova has been confirmed by the method defined in Japan Pharmacopoeia (from product description of BMM).

To the HCII purified fraction obtained in Example 1, which was substantially free of a degrading factor, degraded HCII, polymerized HCII and a coloring substance, was added HCV-positive plasma and the mixture was filtered through a 0.22 $\mu$m membrane (manufactured by Millipore) and the above-mentioned BMM module by a cross flow filtration method. The filtration conditions were: temperature of HCII solution, 4 - 10°C, filtration pressure 0.8 kgf/cm$^2$. The recovery rate of HCII into the filtrate by the filtration step was 97%. After completion of the filtration, the potency of the HCII solution was adjusted and filtration for sterilization was applied. The obtained HCII solution was dispensed to give liquid preparations.

**Experimental Example 1: Confirmation of virus removal effect**

The virus genome amount of hepatitis C in the preparation obtained in Example 6 was measured by PCR.

(1) Extraction of RNA from sample

To this preparation were added guanidine, guanidine thiocyanate and sarcosyl, and phenol/chloroform was added to remove protein. Using isoamyl alcohol, RNA was allowed to form sediment. Each reaction was carried out at room temperature.

(2) Preparation of cDNA by reverse transcriptase

Random hexamer was annealed with the RNA obtained in (1) and using a reverse transcriptase, the RNA was reacted at 42°C for 1 hr to give cDNA.

(3) Amplification of cDNA by Nested PCR

Using HCV primer [from 5' terminal non-coding region of HCV genome, using as a sense primer, 5'-GTGAGTACACCGGAATTGCC-3' (SEQ ID No. 1) and 5'-CACGGTCTACGAGACCTCCC-3' (SEQ ID No. 2), as anti-sense primer, 5'-ACGACCGGGTCCTTTCTTGG-3' (SEQ ID No. 3) and 5'-GCACTCGCAAGCACCCTATC-3' (SEQ ID No. 4)] and Taq polymerase, HCV cDNA was amplified. cDNA was denatured by heating at 94°C for 30 sec and annealed at 55°C for 2 min. cDNA was amplified at 72°C for 2 min. This operation was repeated not less than 20 cycles to give a reaction product.

(4) Analysis of PCR reaction product

The reaction product obtained in (3) was applied to 6% (w/v) polyacrylamide gel electrophoresis (PAGE) using ethidium bromide.

As a result of the experiment, the HCV genome amount in the HCII solution was 103 PCR unit/ml before the filtration, but after the filtration, it decreased to a level below detection limit (1 PCR unit/ml).

**Industrial Applicability**

According to the production method of an HCII-containing preparation of the present invention, a degrading factor is removed from HCII, thus obliterating the decomposition (degradation) during storage of HCII. Consequently, an effective HCII-containing preparation having a higher purity and higher safety can be provided.

Free text of Sequence Listing

**SEQ ID No. 1**

Oligonucleotide designed to act as sense primer for amplifying 5' non-coding region of HCV genome.

**SEQ ID No. 2**

Oligonucleotide designed to act as sense primer for amplifying 5' non-coding region of HCV genome.

**SEQ ID No. 3**

Oligonucleotide designed to act as antisense primer for amplifying 5' non-coding region of HCV genome.

**SEQ ID No. 4**

Oligonucleotide designed to act as antisense primer for amplifying 5' non-coding region of HCV genome.

This application is based on a patent application No. 320349/1997 filed in Japan, the content of which is hereby incorporated by reference.

In addition, all publications inclusive of specifications of patents and patent applications are herein incorporated by reference to the same extent as if each individual publication was specifically described herein.

**Claims**

1. A heparin cofactor II-containing preparation substantially free of at least one contaminant selected from a group consisting of a degrading factor, degraded heparin cofactor II, polymerized heparin cofactor II and a coloring substance.

2. A heparin cofactor II-containing preparation substantially free of a degrading factor and degraded heparin cofactor II.

3. The heparin cofactor II-containing preparation of claim 2, which is further substantially free of polymerized heparin cofactor II and/or a coloring substance.

4. A heparin cofactor II-containing preparation comprising heparin cofactor II having a purity of not less than 98%.

5. The heparin cofactor II-containing preparation of any of claims 1 to 4, which is substantially free of an infective virus.

6. A production method of a heparin cofactor II-containing preparation, comprising a step of separating heparin cofactor II and a degrading factor from a solution containing the heparin cofactor II and the degrading factor.

7. The method of claim 6, wherein the step comprises one or more treatment(s) selected from the group consisting of hydrophobic (interaction) chromatography, fractionation by a water soluble polymer, salting out and affinity chromatography using a basic amino acid as a ligand.

8. The method of claim 6 or 7, further comprising a step for removing degraded heparin cofactor II and/or a coloring substance.

9. The method of claim 8, wherein the step is gel filtration chromatography.

10. The method of any of claims 6 to 9, further comprising at least one step for virus removal or virus inactivation, which is selected from the group consisting of filtration, a heating treatment and a surfactant treatment.

11. The method of claim 10, wherein the step is filtration using a porous hollow fiber.

FIG.1

# FIG. 2

FIG. 3

# INTERNATIONAL SEARCH REPORT

| | International application No. |
|---|---|
| | PCT/JP98/04939 |

## A. CLASSIFICATION OF SUBJECT MATTER
Int.Cl⁶ A61K38/00-38/58, C07K14/47, C07K14/745, C07K14/81, C07K1/14-1/36

According to International Patent Classification (IPC) or to both national classification and IPC

## B. FIELDS SEARCHED

Minimum documentation searched (classification system followed by classification symbols)
Int.Cl⁶ A61K38/00-38/58, C07K14/47, C07K14/745, C07K14/81, C07K1/14-1/36

Documentation searched other than minimum documentation to the extent that such documents are included in the fields searched

Electronic data base consulted during the international search (name of data base and, where practicable, search terms used)
CAPLUS (STN), REGISTRY (STN)

## C. DOCUMENTS CONSIDERED TO BE RELEVANT

| Category* | Citation of document, with indication, where appropriate, of the relevant passages | Relevant to claim No. |
|---|---|---|
| X<br>Y | JP, 9-286797, A (The Green Cross Corp.),<br>4 November, 1997 (04. 11. 97),<br>Refer to Par. Nos. [0016] to [0021], [0031] to [0034]<br>(Family: none) | 1-10<br>11 |
| X<br>Y | TOULON, P. et al., "Purification of heparin cofactor II from human plasma" J. Chromatogr., 1991, Vol. 539, No. 2, p.493-500, refer to ABSTRACT, *Purification of HCII*, TABLE I | 1-4, 6-9<br>5, 10, 11 |
| X<br>Y | PETZELBAUER, E. et al., "MODULATION OF HEPARIN COFACTOR II ACTIVITY BY GLYCOSAMINOGLYCANS AND ADHESIVE GLYCOPROTEINS" Thromb. Res., 1992, Vol. 66, p.559-567, refer to ABSTRACT, MATERIALS AND METHODS, Fig. 1 | 1-4, 6, 7<br>8-11 |

[X] Further documents are listed in the continuation of Box C.    [ ] See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 17 February, 1999 (17. 02. 99) | 2 March, 1999 (02. 03. 99) |

| Name and mailing address of the ISA/ | Authorized officer |
|---|---|
|     Japanese Patent Office | |
| Facsimile No. | Telephone No. |

Form PCT/ISA/210 (second sheet) (July 1992)

## INTERNATIONAL SEARCH REPORT

International application No.

PCT/JP98/04939

C (Continuation).  DOCUMENTS CONSIDERED TO BE RELEVANT

| Category* | Citation of document, with indication, where appropriate, of the relevant passages | Relevant to claim No. |
|---|---|---|
| X<br>Y | YAMAGISHI, R., et al., "PURIFICATION AND BIOLOGICAL PROPERTY OF HEPARIN COFACTOR II: ACTIVATION OF HEPARIN COFACTOR II AND ANTITHROMBIN III BY DEXTRAN SULFATE AND VARIOUS GLYCOSAMINOLYCANS" Thromb. Res., 1984, Vol. 36, p.633-642, MATERIALS AND METHODS (Purification OF HCII), refer to TABLE 1 | 1-4, 6, 7<br>8-11 |
| Y | JP, 2-167232, A  (Asahi Chemical Industry Co., Ltd.), 27 June, 1990 (27. 06. 90), Refer to Claims  (Family: none) | 11 |
| A | JP, 3-128398, A  (The Green Cross Corp.), 31 May, 1991 (31. 05. 91), Refer to the full text & EP, 408029, A1 & US, 5138034, A | 1-4, 6, 7 |
| A | EP, 416983, A1 (CENTRE REGIONAL DE TRANSFUSION SANGUINE DE LILLE), 13 March, 1991 (13. 03. 91), Refer to the full text & JP, 4-124199, A & US, 5679776, A | 1-4, 6, 7 |
| A | US, 5219995, A (ALPHA THERAPEUTIC CORPORATION), 15 June, 1993 (15. 06. 93), Refer to the full text & WO, 94/01466, A1 & EP, 607392, A1 & JP, 6-511018, A | 1-4, 6, 7 |
| A | EP, 496725, A2 (IMMUNO AKTIENGESELLSCHAFT), 29 July, 1992 (29. 07. 92), Refer to the full text & US, 5281661, A & US, 5409990, A & JP, 4-295432, A | 1-4, 6, 7 |

Form PCT/ISA/210 (continuation of second sheet) (July 1992)